# EUROPEAN PATENT APPLICATION

(11) **EP 1 176 134 A1**
(43) Date of publication of application: **30.01.2002**
(21) Application number: 00908027.6
(22) Date of filing: 13.03.2000
(51) Int. Cl.: C07C 59/92, C07C 69/738, C07C 403/20, C07C 403/24, C07D 213/79, C07D 213/80, C07D 307/32, C07D 307/58, A61K 31/12, A61K 31/122, A61K 31/192, A61K 31/216, A61K 31/341, A61K 31/455, A61P 3/10, A61P 9/10, A61P 9/12, A61P 35/00, A61P 37/00, A61P 37/06, C07K 14/765

(54) **LIGANDS OF NUCLEAR RECEPTORS PPAR'S**

(30) Priority: 11.03.1999 JP 10699699
(71) Applicant: Nuclear Receptor Research Limited, Kawasaki-shi, Kanagawa 213-0033 (JP); Institute of Research and Innovation, Tokyo 113-0034 (JP)
(72) Inventor: TAMURA, Gakuzo, Ohta-ku, Tokyo 143-0023 (JP); ANDO, Kunio, Kawasaki-shi, Kanagawa 213-0033 (JP); MAGAE, Junji, Toride-shi, Ibaraki 302-0034 (JP)
(74) Representative: Reinhard - Skuhra - Weise & Partner
(86) International application number: JP0001497
(87) International publication number: WO0053563

(57) **Abstract**

Ligands of nuclear receptors PPAR's (peroxisome proliferator-activated receptors) which are compounds selected from the group consisting of ascofuranone; and ascofuranone homologs and ascochlorin homologs having at least an orcyl aldehyde moiety wherein the hydrogen atom(s) of the hydroxyl group(s) at the 2-position and/or the 4-position of the orcyl aldehyde moiety are substituted by C₁₋₁₅alkyl, C₁₋₁₅alkenyl, CH₂COOH, CH₂COO (C₁₋₁₅alkyl), C(C=O)(C₁₋₁₅ alkyl), C(=O)(CH₂)₁₋₁₅COOH, nicotinoyl, isonicotinoyl, etc. These ligands are usable in preventing and/or treating diabetes; hypertension or cerebrovascular disorders; arteriosclerosis; complications of diabetes; chronic inflammation; cachexia; digestive cancers, etc.

## Description

### FIELD OF THE INVENTION

The present invention relates to ligands for the nuclear receptor, PPAR, and treatment and/or prevention of diseases capable of being cured by the PPAR ligand-dependent regulation of gene expression.

### PRIOR ART

The present invention finds industrial applications in the field of medical products and encompasses candidate compounds for medical products for treating metabolic diseases, chronic inflammation and malignant tumors in animals including human. Steroids, retinoids, active vitamin D3 and thyroid hormones show marked physiological activity for animals in a very small amount. These are collectively called fat-soluble hormones because they dissolve in organic solvents but not in water. With the progress of research on the mechanism by which fat-soluble hormones show specific activity in a very small amount, similaritities in their action mechanisms have been shown. These fat-soluble hormones produce a physiological effect by binding their receptors present in the cytoplasm and then binding a specific site of a gene to directly control the expression of genetic information. Analyses of DNA encoding nuclear receptors showed surprising homology in the structure of nuclear receptors. In order for these fat-soluble hormones to act on animal cells, they must penetrate the cell membrane to bind nuclear receptors present in the cytoplasm. Nuclear receptors bound to the hormones pass through the nuclear membrane to enter the nucleus where they bind the hormone recognition site of the chromosome as a dimer formed of two receptors to control the expression of genetic information.

In order for a nuclear receptor bound to a fat-soluble hormone to control the expression of genetic information, it must be combined with another receptor to form a dimer. Dimers include homodimers formed of identical receptors and heterodimers formed of different receptors. Steroids such as sex hormones and adrenal cortical hormones act as homodimers formed of identical receptors. On the contrary, many fat-soluble hormones act as heterodimers. However, not every combination of receptors is available as a heterodimer. Most of them form a heterodimer with a nuclear receptor called retinoid X receptor (RXR) to bind the recognition site of the chromosome. The reason why these fat-soluble hormone receptors must be combined with retinoid X receptor to produce their effect has not been well known.

On the other hand, many pharmaceutical companies have researched and developed drugs for treating and/or preventing coronary arteriosclerosis. Sclerotic lesion of the coronary artery is the leading cause of death in developed countries and one of the most critical inducing factors thereof is hyperlipoproteinemia associated with an excessive level of low-density lipoprotein (LDL) in the blood. Hyperlipoproteinemia can also be called hypercholesterolemia because LDL is rich in cholesterol. With the aim of treating and/or preventing coronary arteriosclerosis, serum hypolipidemic agents for treating hyperlipoproteinemia have been sought for many years. In 1960s, a serum hypolipidemic agent called clofibrate first appeared. Clofibrate was abundantly used for not only patients of coronary arteriosclerosis but also patients of cerebral arteriosclerosis, intermittent claudication among symptoms of femoral arteriosclerosis or type II diabetes liable to induce coronary arteriosclerosis and achieved a temporary great commercial success though its action mechanism remained unclear. However, it was gradually abolished because of two disadvantages. First, it acts to decrease very low-density lipoprotein (VLDL) rich in serum neutral lipid rather than decrease serum total cholesterol. Second, its pharmaceutical efficacy was not always reliable as several double blind tests on many patients of coronary arteriosclerosis showed that clofibrate induced death by heart infarction more frequently than a placebo, was no different from a placebo or frequently produced side effects. However, clinicians who administered clofibrate to patients brought important information on action mechanism of the drug.

Patients of type II diabetes are said to suffer from vascular disorder ten times more frequent and have a life about ten years shorter than the average person. Thus, clofibrate was administered to many patients of type II diabetes to prevent vascular disorder. However, hypoglycemic coma occurred at a high frequency among patients of type II diabetes who received clofibrate in combination with a hypoglycemic sulfonylurea drug. Hypoglycemic coma should not naturally occur in patients of type II diabetes associated with sustained hyperglycemic state. Examination of cases of coma showed that Langerhans' β cells stimulated by the sulfonylurea drug released a considerable amount of insulin while clofibrate reduced insulin resistance in peripheral tissue, whereby blood sugar rapidly decreased via synergism. That is, clofibrate and its derivatives have been known to possibly reduce insulin resistance in peripheral tissue.

In 1982, the inventors reported that an ascochlorin derivative AS-6 reduces blood sugar and insulin in normal and streptozotocin-treated rodents (Agr. Biol. Chem. 46: 2865-69, 1982). In late 1980, several pharmaceutical companies started research and development of drugs for improving insulin resistance characteristic of type II diabetes. A model compound for designing candidate compounds was ciglitazone of Takeda Chemical Industries, Ltd., which is a compound combining clofibrate capable of enhancing insulin sensitivity in peripheral tissue (an allyloxy compound) with thiazolidine producing a hypoglycemic effect. Although it failed in development trials due to the lack of efficacy, it should be highly valued as a model compound of later thiazolidinedione compounds (TZDs). After subsequent development of TZD drugs such as troglitazone, pioglitazone and rosiglitazone, the treatment of type II diabetes reached a turning point with the appearance of the TZDs whose main efficacy lay in the rescission of insulin resistance. The active mechanism of TZDs was not initially known, but research into clofibrate triggered an explanation. It was discovered that a nuclear receptor whose ligand is unknown (orphan receptor) PPARα is activated by clofibrate, suggesting that TZDs may also control the expression of genetic information as ligands for PPAR. PPAR is an abbreviation of peroxisome proliferator-activated receptor named after clofibrate because it brings about growth of peroxisome of the liver to induce liver hypertrophy. As a matter of course, TZDs partially sharing a chemical structure with clofibrate were also examined for interaction with PPAR receptors. The results showed that TZDs are targeted at white adipose cells so that TZDs are ligands activating nuclear receptor PPARγ highly expressed in white adipose cells. This finding led to correction of the hypothesis about the etiology of type II diabetes. The previous hypothesis was that white adipose tissue is a silent tissue storing fat and that it lowered insulin sensitivity of type II diabetes patients resulting from the expression of insulin resistance in the liver or muscle.

Since then, white adipose tissue has been considered as a target organ for preventing/treating many diseases called life style-related diseases and many findings on molecular level about differentiation of adipose cells and control mechanism thereof have accumulated. At present, it is known that PPARγ is highly expressed in white adipose tissue, lymphatic tissue, adrenal gland and intestines. In adipose tissue, PPARγ was shown to play an important role in differentiation of adipose cells in conjugation with cofactors of PPARγ (CBP and similar molecules) and that dietary fatty acids such as linolic acid or arachidonic acid are ligands for the PPAR family, thus increasing the importance of the PPAR family as a target of diet therapy or medical therapy. Explanation of the molecular mechanism of differentiation of adipose cells focused on PPAR-CBP partially elucidated the control mechanism of energy metabolism in adipose tissue, which raised expectations for finding a target of novel therapy or a drug for life style-related diseases based on obesity. PPAR includes 3 classes among which PPARα is mainly distributed in hepatic cells, PPARβ is systemically distributed and PPARγ is distributed in adipose cells, the adrenal gland and intestines. Adipose cells thickened by a manydeposits of neutral fat due to overeating produce a group of cytokines called adipocytokines and release them into circulating blood. It has been gradually established that these adipocytokines decrease insulin sensitivity in peripheral tissue. It is noteworthy that adipocytokines include TNF-α, which was shown to greatly inhibit glucose uptake in peripheral tissue to induce cachexia in terminal cancer patients when administered to animals.

On the other hand, PPARγ expressed in lymphatic tissue is known to also be highly expressed in the spleen, intestines and the adrenal gland in addition to adipose tissue and research on the role of PPARγ expressed in these organs has advanced. Especially, two prominent papers about the role of PPARγ expressed in lymphatic tissue were published in 1998 (M. Ricote et al., NATURE 391: 79-82, 1998; C. Jiang, A.T. Ting and B. Seed, NATURE 391: 82-86, 1998). These focused on the relation of PPARγ to chronic inflammation. Activated macrophages infiltratedinflammation sites to actively biosynthesize inflammatory cytokines such as TNF-α, IL-1β and IL-6 so that inflammation becomes chronic. Said papers showed that, when a non-steroid analgesic/antiphlogistic which is a ligand for PPARγ is added to cultures of activated macrophages, the transcription of genes associated with inflammatory cytokine production is dose-dependently suppressed and that the non-steroid analgesic/antiphlogistic agent suppresses the expression of inflammatory cytokine genes by partially competing with transcription accelerator factors AP-1, STAT and NF-κ. Thus, non-steroid analgesics/antiphlogistics suppress transcription of inflammatory cytokine genes such as IL-1β, TNFα and IL-6 into messenger RNA by activating nuclear receptor PPARγ, thereby inhibiting biosynthesis of inflammatory cytokines. Existing TZD compounds cannot be expected to have any application in the treatment of chronic inflammation because they scarcely suppress inflammatory cytokine production of activated macrophages. However, the suppression of inflammatory cytokine production by PPARγ ligands may possibly lead to the treatment of chronic inflammation associated with overexpression of PPARs such as lesions of arteriosclerosis, diabetic complication and chronic rheumatoid arthritis as well as malignant tumor.

The action mechanism of non-steroid analgesics/antiphlogistics lies in the suppression of production of prostaglandin, which brings about acute pain and enlargement at inflammatory sites. However, release of inflammatory cytokines such as TNFα is as important to prostaglandin production during the process where inflammation becomes chronic, as shown by the fact that systemic administration of a monoclonal antibody against TNFα or IL-6 receptors improves chronic inflammation such as that associated with chronic rheumatoid arthritis. Non-steroid analgesics/antiphlogistics used for the treatment of acute inflammation are difficult to use for a long period because they inhibit prostaglandin biosynthesis in gastrointestinal mucosa, thus causing peptic ulcer at high frequency. For the treatment of chronic inflammation, it is desirable to develop a drug that suppresses inflammatory cytokine production but not prostaglandin biosynthesis. Recently, Ross's theory has been widely accepted, stressing that coronary arteriosclerosis is a typical chronic inflammation similar to chronic hepatitis, glomerular nephritis, chronic pancreatitis and chronic rheumatoid arthritis (R. Ross, New Eng. J. Medicine 340 :115-126, 1999). Thus, the therapy whereby inflammatory cytokine production is suppressed by activated macrophages has the potential in the treatment of intractable diseases associated with chronic inflammation associated with coronary arteriosclerosis, chronic hepatitis, glomerular nephritis, chronic pancreatitis and chronic rheumatoid arthritis.

Some malignant tumors produces many TNF, thus inviting metabolic decay called cachexia in patients. Patients suffering with cachexia cannot assimilate intake energy and rapidly weaken with the energy balance turned negative. At the same time, they lose immune potency to allow growth of opportunistic pathogens so that they often die of sepsis. Seventy percent of cancer patients die of opportunistic pathogen-induced sepsis rather than cancer. Therefore, it is essential to control TNFα production in malignant tumors for prolonging the life of cancer patients. Interestingly, TNFα production is not controlled at all in colon cancer cells, which express substantial amounts of PPARγ. This means that PPARs expressed in colon cancer cells cannot control TNFα production because they are not activated by their ligands. This is proved by the fact that patients of colon cancer treated with a ligand for PPARγ Sulindac temporarily show signs of remission by suppression of TNFα production. As a matter of course, the non-steroid analgesic/antiphlogistic agent Sulindac is difficult to administer for a long period to inhibit biosynthesis of prostaglandin. Such a fact demonstrates that high expression of nuclear receptors in cells alone is insufficient for the nuclear receptors to control transcription of genetic information but a necessary and sufficient amount of a specific ligand should be externally supplied. Thus, PPARγ ligands may possibly play an important role in treating and prolonging the life of patients suffering with gastrointestinal cancer.

Chronic inflammation associated with chronic rheumatoid arthritis is treated with drugs used in the treatment of acute inflammation, namely non-steroid analgesics/antiphlogistics or glucocorticoids; and it can be said that no specific therapeutic agent for chronic inflammation has existed. Non-steroid analgesics/antiphlogistics have the disadvantage that chronic use often causes side effects on the digestive system such as peptic ulcers because they inhibit biosynthesis of prostaglandin. Glucocorticoids also have many side effects such as the inhibition of prostaglandin biosynthesis, frequent incidence of infectious diseases due to immunosuppression, elicitation of metabolic disorder such as diabetes, rebound phenomenon, which means that drug withdrawal aggravates the condition rather than before medication. Therefore, it cannot be said that current treatments for chronic inflammation relying on these drugs remarkably improves the quality of life (QOL) of patients. Further attention should be paid to diabetic complications with a background of microvascular inflammation. If a drug capable of specifically curing chronic inflammation were developed, it could also probably prevent/treat diabetic complication caused by chronic vascular inflammation. It is highly desirable to produce a drug capable of treating chronic vascular inflammation associated with chronic rheumatoid arthritis, arteriosclerosis, diabetic microangiopathy, periarteritis nodosa and aneurysms without QOL deterioration due to frequent side effects. In future, ligands for PPARγ will be the object of active research on antiphlogistics against chronic inflammation.

The focus of the researchers' attention was what kind of effect on metabolic disorder of type II diabetes is shown by ligands for nuclear receptor RXR that forms a heterodimer with PPARγ to control the expression of genetic information. The first study to shed some light onthis issue was published in 1997 (Nature 386: 407-410, 1997). This study shows the results of cell culture and animal experiments involving retinoid derivatives LG100268 and LGD1069 being developed as therapeutic agents for promyelocytic leukemia, Kaposi's sarcoma or the like and reports that (1) these derivatives control the expression of genetic information in cell culture, (2) they enhance insulin sensitivity to dramatically decrease blood sugar, serum neutral fat and serum insulin when they are orally administered to hereditary obese and diabetic mice ob/ob and db/db, and (3) RXR ligands and PPARγ ligands show synergism in vivo and in vitro when small amounts of the retinoid derivatives and TZD, each having no efficacy alone, are administered in combination. However, the possibility that the same compound acts as a ligand for both RXR and PPAR receptors has been neither referred to nor reported.

### SUMMARY OF THE INVENTION

Previously, the inventors carried out studies to find effective means for treating and/or preventing metabolic diseases and chronic inflammation and filed a patent application for a ligand binding a retinoid X receptor selected from the group consisting of ascochlorin and its analogous compounds and derivatives thereof (JPA No. 377905/98 and International Application PCT/JP/07012).

The inventors' further studies revealed that ascochlorin and ascofuranone and derivatives thereof bind PPARγ receptor to control gene expression and that a derivative of an RXR ligand ascochlorin included in the prior patent application in which the hydroxyl group at the 2-position and/or 4-position has been substituted also binds PPARγ receptor to control gene expression. It is a novel finding that such compounds act as ligands for two nuclear receptors at the same time.

Accordingly, the present invention provides nuclear receptor PPAR ligands, which are ascofuranone, ascofuranone homologues or ascochlorin homologs. They can be used to treat and/or prevent diseases or conditions which can be alleviated by the PPAR ligand-dependent regulation of the transcription of genetic information in mammals, more specifically to treat and/or prevent diabetes including type II diabetes induced by the expression of insulin resistance; hypertension or cerebral angiopathy; arteriosclerosis including coronary arteriosclerosis; diabetic complication; chronic inflammation including chronic inflammation of organs including vessel, liver, kidney, joints, intestines and pancreas and chronic inflammation associated with autoimmune diseases; cachexia occurring in terminal cancer patients; gastrointestinal cancer including colon cancer, stomach cancer and liver cancer; and to prevent degeneration and/or necrosis of β cells in the pancreatic islets of Langerhans.

### BRIEF DESCRIPTION OF THE DRAWINGS

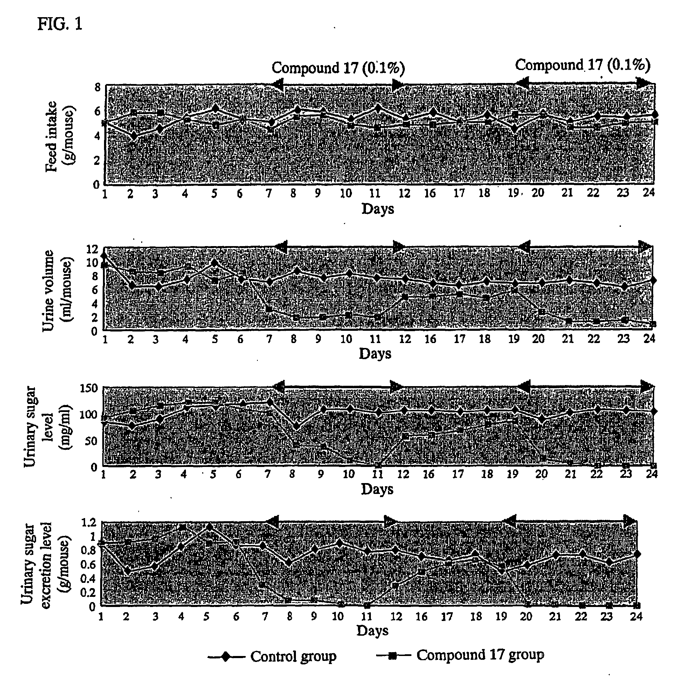

FIG. 1 shows the effects of compound 17 that improves polydipsia/polyuria and inhibits urinary sugar excretion in hereditary obese and diabetic mice (db/db mice).

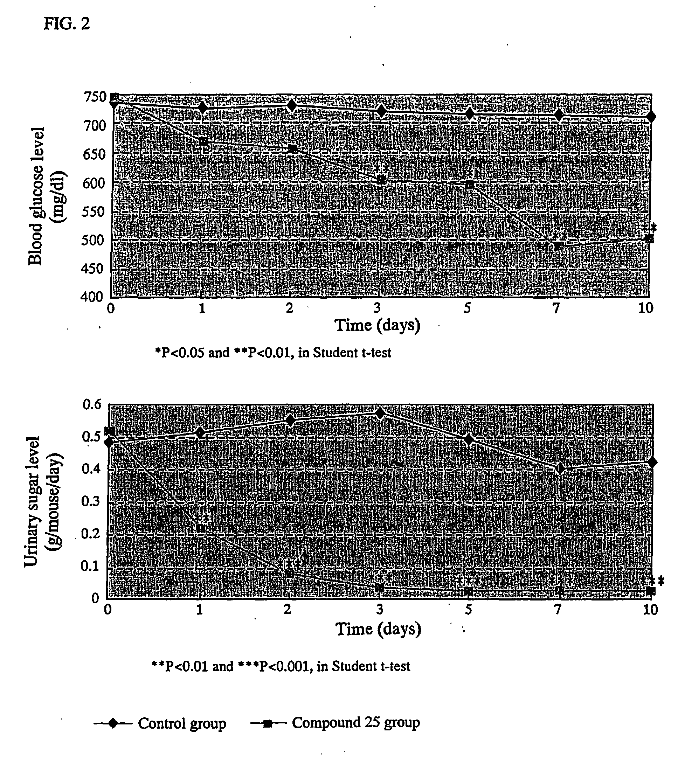

FIG. 2 shows changes in blood glucose level and urinary sugar excretion in db/db mice treated with compound 25. Blood was collected from the tail vein of ten db/db mice at 12 weeks of age 2 days before the start of experiments, and the animals were divided into two groups each having an average blood glucose level of 720-740 mg/dl. One group was a control group, while the other group orally received compound 25 at 60 mg/kg once daily. A solid diamond denotes the control group while the hatched square denotes the group treated with compound 25. **P < 0.01 and ***P < 0.001 in Student t-test.

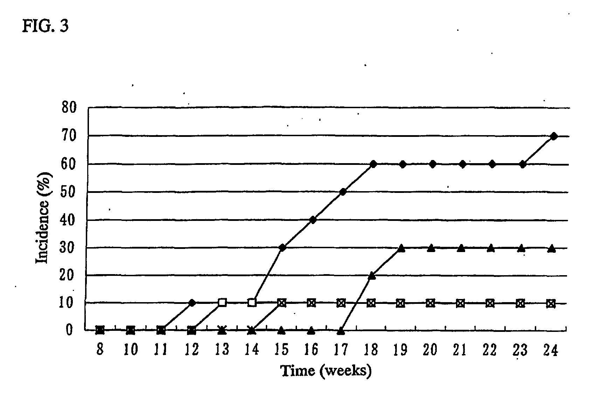

FIG. 3 shows the effects of compound 1 and compound 25 that prevent onset of type I diabetes in NOD mice. SA solid diamond denotes the control group, solid triangle denotes the: group treated with compound 1 at 25 mg/kg, open square denotes the: group treated with compound 1 at 50 mg/kg, and cross denotes the: group treated with compound 25 at 50 mg mg/kg. Each group consists of 10 animals. The incidence of type I diabetes at week 24 was 70% in the control group, 30% in the group treated with compound 1 at 25 mg/kg and 10% in the groups treated with compounds 1 and 25 at 50 mg/kg, and the incidence rates of the test showed a significant difference of 5% between the latter two groups and the control group.

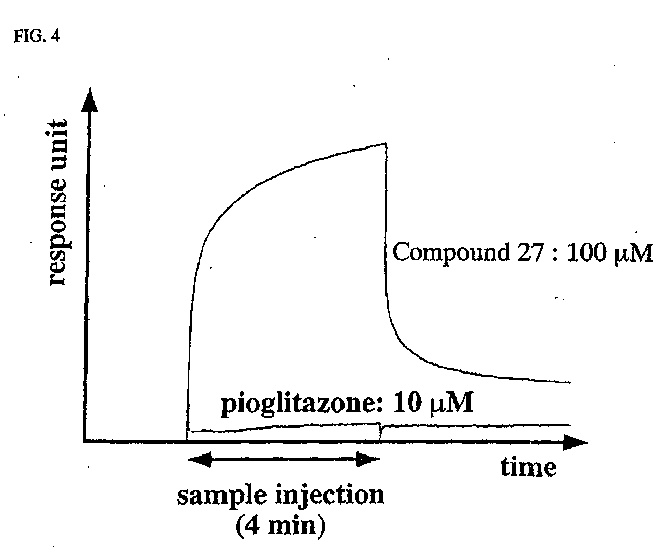

FIG. 4 shows the results of binding tests of compound 27 and pioglitazone using a Surface Plasmon Resonance sensor. Pioglitazone generates no signal because it is non-binding, but compound 27 clearly generates a signal.

### DISCLOSURE OF THE INVENTION

The present invention provides a nuclear receptor PPAR ligand, which is a compound selected from the group consisting of ascofuranone; and ascofuranone homologues or ascochlorin homologues having at least an orcylaldehyde moiety wherein the hydrogen of the hydroxyl group at the 2-position and/or 4-position of the orcylaldehyde moiety is substituted by a group selected from the group consisting of -(C₁-C₁₅)alkyl or alkenyl, aryl, (C₁-C₁₅)alkylaryl, -CH₂COOH, -CH₂COO(C₁-C₁₅)alkyl, -C(=O)(C₁-C₁₅)alkyl, -C(=O)aryl, -C(=O)(C₁-C₁₅)alkylaryl, -C(=O)(CH₂)₁₋₁₅COOH, nicotinoyl or isonicotinoyl. As used herein, "ascofuranone homologs" mean, except in special cases, compounds having a structure similar to that of ascofuranone having at least an orcylaldehyde moiety wherein an appropriate side chain (eg, terpenoid) is attached at the 3-position while the 5-position may be substituted by Cl or H like ascofuranone and capable of binding nuclear receptors PPAR, and include dehydroascofuranone. As used herein, "ascochlorin homologs" mean, except for special cases, compounds having a structure similar to that of ascochlorin having at least an orcylaldehyde moiety wherein an appropriate side chain (eg, terpenoid) is attached at the 3-position while the 5-position may be substituted by Cl or H like ascochlorin (otherwise referred to as ascochlorin analogous compounds) and capable of binding nuclear receptors PPAR. The present invention also provides a nuclear receptor PPAR ligand, which is a compound represented by the formula: 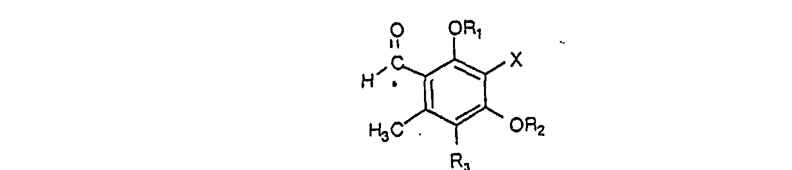 wherein x is 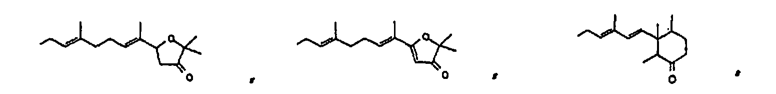 or 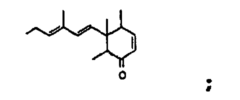 R₁ is H, -(C₁-C₅)alkyl or alkenyl or -C(=O)(C₁-C₅)alkyl; R₂ is H, -(C₁-C₅)alkyl or alkenyl, -CH₂COOH, -CH₂COO(C₁-C₅)alkyl, -C(=O)(C₁-C₅)alkyl, -C(=O)(CH₂)₁-₅COOH, nicotinoyl or isonicotinoyl; and R₃ is H or Cl, provided that ascochlorin is excluded.

### PPAR ligands of the present invention include:

-ascofuranone and derivatives thereof represented by formula (1):

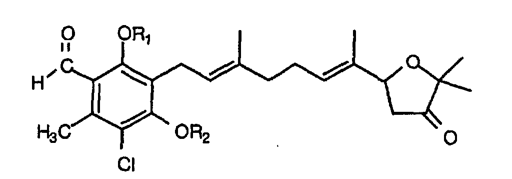

**Table 1.**

| Ascofuranone and derivatives thereof | | | |
|---|---|---|---|
| Compound # | R₁ | R₂ | Note |
| 1 | H | H | Ascofuranone (AF) |
| 2 | H | -COCH₃ | 4-O-acetyl derivative |
| 3 | H | -CH₃ | 4-O-methyl AF |
| 4 | -CH₃ | -CH₃ | 2,4-dimethyl AF |
| 5 | -COCH₃ | -CH₃ | 4-O-metyl, 2-0-acetyl AF |
| 6 | -CH₃ | -COCH₃ | 2-O-methyl, 4-0-acetyl AF |
| 7 | -CH₃ | H | 2-O-methyl AF, novel compound |
| 8 | H | -CH₂CH₃ | 4-O-ethyl AF |
| 9 | H | -allyl | r-O-allyl AF |
| 10 | H | -butyl | 4-O-butyl AF |
| 11 | H | -CH₂COOH | |
| 12 | H | -CH₂COOCH₃ | |
| 13 | H | -nicotinoyl | |
| 14 | H | -isonicotinoyl | Novel compound |
| 15 | H | -CO(CH₂)₃COOH | Novel compound |

-ascochlorin and derivatives thereof represented by formula (2):

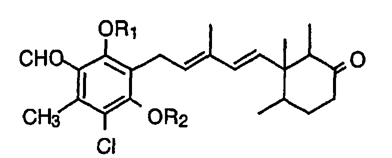

**Table 2.**

| Compound # | R₁ | R₂ | Note |
|---|---|---|---|
| 16 | H | H | Ascochlorin (AS) |
| 17 | H | -CH₃ | 4-O-MeAS |
| 18 | -CH₃ | -CH₃ | 2,4-diMeAS |
| 19 | -COCH₃ | -CH₃ | |
| 20 | -CH₃ | -COCH₃ | |
| 21 | -CH₃ | H | |
| 22 | H | -CH₂CH₃ | |
| 23 | H | -allyl | |
| 24 | H | -butyl | |
| 25 | H | -CH₂COOH | |
| 26 | H | -nicotinoyl | |
| 27 | -CH₃ | -CH₂COOH | Novel compound |
| 28 | -CH₃ | -CH₂COOCH₂CH₃ | Novel compound |
| 29 | H | -CO(CH₂)₃COOH | Novel compound |
| 30 | H | -isonicotinoyl | Novel compound |
| 31 | -CH₃ | -isonicotinoyl | Novel compound |

-ascochlorin analogous compounds represented by formula (3) :

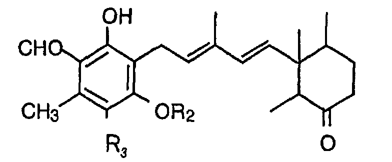

**Table 3.**

| Ascochlorin analogous compounds | | |
|---|---|---|
| Compound # | R₂ | R₃ |
| 32 | H | H |
| 33 | -CH₃ | H |
| 34 | -COCH₃ | H |

and formula (4):

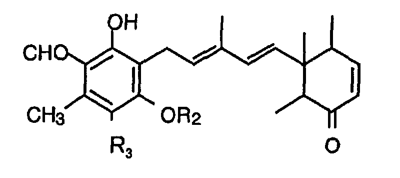

**Table 4.**

| Ascochlorin analogous compounds | | |
|---|---|---|
| Compound # | R₂ | R₃ |
| 35 | H | H |
| 36 | -CH₃ | Cl |
| 37 | -COCH₃ | Cl |
| 38 | -CH₃ | H |
| 39 | -COCH₃ | H |

- dehydroascofuranone and derivatives thereof represented by formula (5): 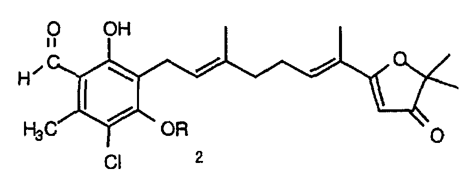 wherein H of the hydroxyl group at the 2-position of the orcylaldehyde may be substituted by -(C₁-C₅)alkyl or alkenyl or -C(=O)(C₁-C₅)alkyl; R₂ is H, -(C₁-C₅)alkyl or alkenyl, -CH₂COOH, -CH₂COO(C₁-C₅)alkyl, -C(=O)(C₁-C₅)alkyl,-C(=O)(CH₂)₁₋₅COOH, nicotinoyl or isonicotinoyl; and Cl at the 5-position may be H. Such compounds are novel.

Compounds of the present invention are characterized by the chemical structure in which a terpenoid side chain is attached to the orcylaldehyde group. They are biologically characterized in that they allow a PPAR receptor-binding site-coupled transfer gene to be dose-dependently expressed in mammal cells.

### Preparation process

Compounds of the present invention are mainly grouped into metabolites ascochlorin, ascofuranone and analogous compounds thereof produced by culturing filamentous fungi such as Ascochyta viciae; and derivatives obtained by organochemically modifying them. Ascofuranone is an antibiotic produced by a filamentous fungus Ascochyta viciae reported by one of the inventors in 1972 (Tetrahedron Letters No. 25, 2541-2544); this fungus also produces ascochlorin. Ascochlorin is also reported to be produced by filamentous fungi other than Ascochyta viciae such as Nectria coccinea, Fusarium sp., Cylindocarpon lucidium, Cylindrocladium ilicicola, Cylindrocladium sp., Verticillium sp., etc., but no report has shown that ascofuranone was isolated from these filamentous fungi. Ascofuranone is a metabolite specific to Ascocyta viciae.

Dehydroascofuranone can be synthesized from ascofuranone by dehydrogenating the tetrahydrofurane ring by a standard procedure. Acylated or alkylated dehydroascochlorin can be synthesized as follows. The procedure below can also be used to acylate or alkylate ascochlorin and ascofuranone and derivatives thereof.

As used herein, "aryl" means an atomic group obtained by removing a hydrogen atom from an aromatic hydrocarbon, such as phenyl, tolyl, xylyl, biphenyl, naphthyl, anthryl and phenanthryl which may be substituted by 1 to 3 substituents. Said substituents include (C₁-C₅)alkyl or alkenyl and halo including chloro or nitro.

### Acylation

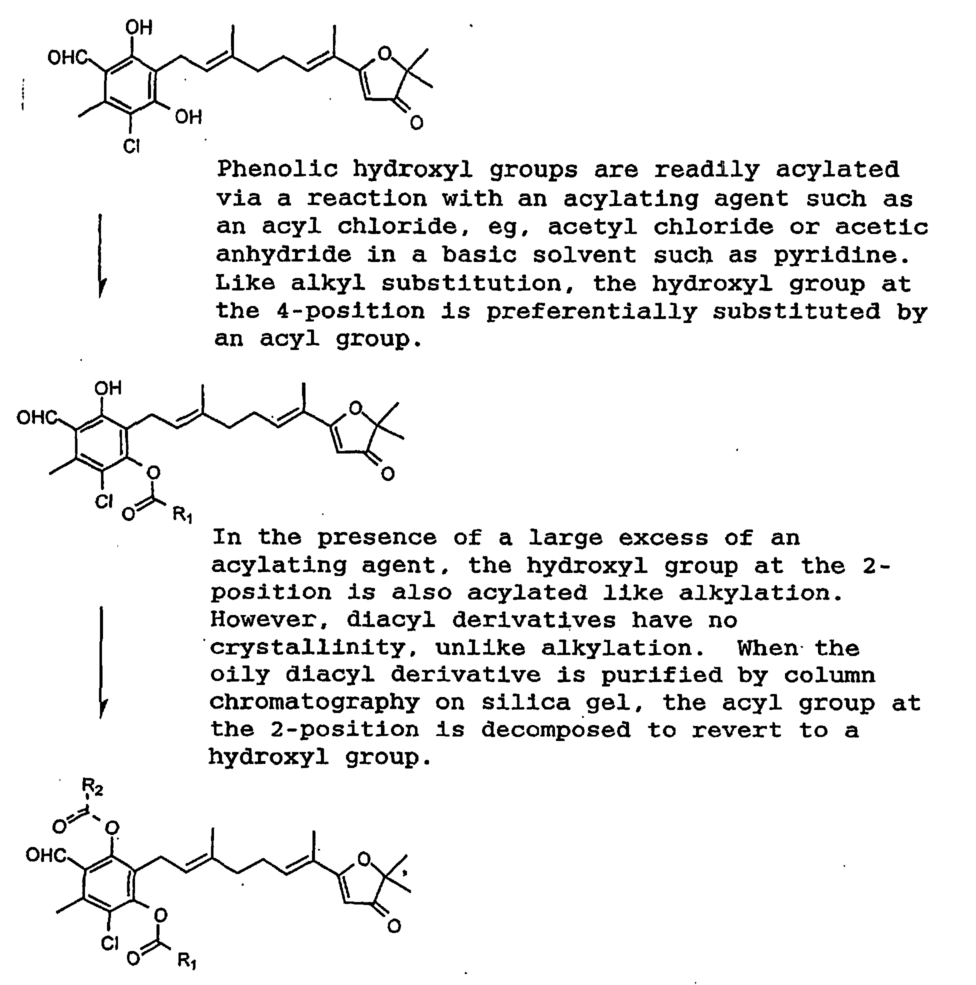

### Alkylation

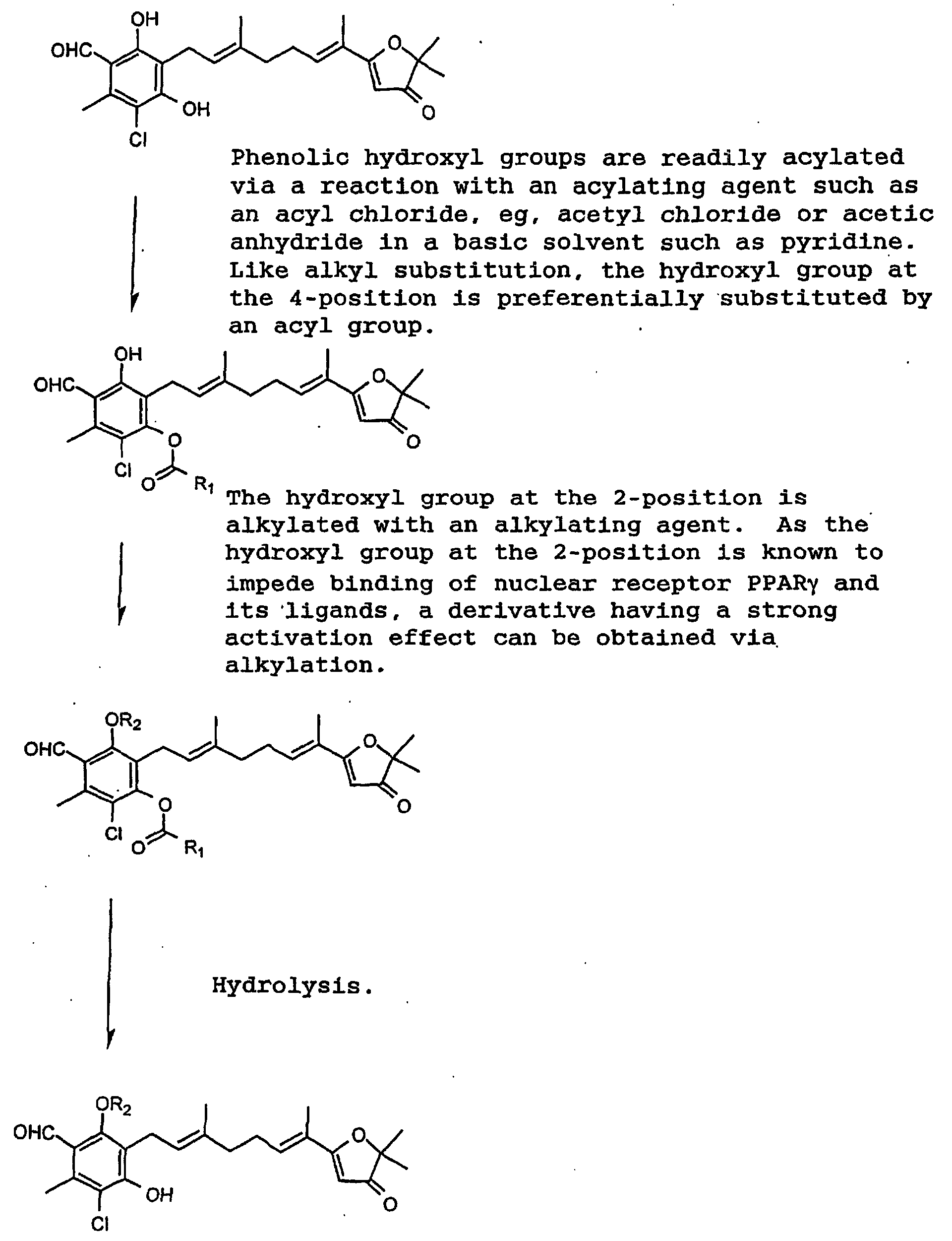

### 2-O-Alkyldehydroascofuranone derivatives

X-ray analysis of PPARγ receptor bound to ascochrolin and ascofuranone derivatives showed that the hydroxyl group at the 2-position of the orcylaldehyde downwardly regulates interaction between the receptor protein and the ligands. Therefore, 2-0-methyl derivatives alkylated at the 2-position hydroxyl group activate PPARγ receptor more strongly than mother compounds. The hydroxyl group at the 2-position is substituted by an alkyl group as follows.

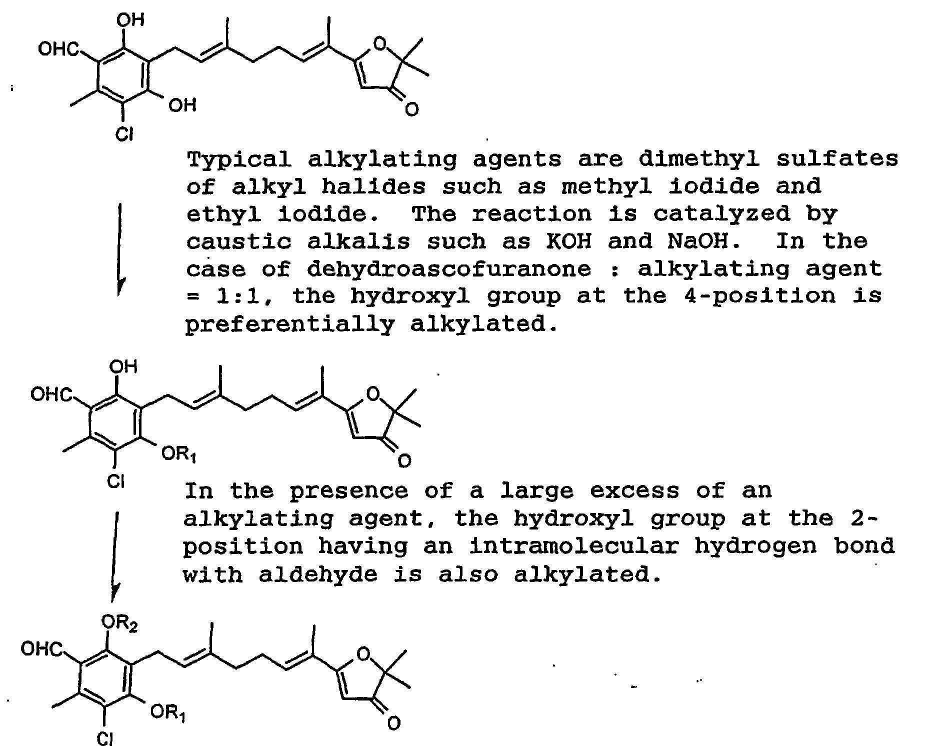

Alkyl groups that can be substituted for crystallization include methyl, ethyl, propyl, isopropyl, butyl, isobutyl, t-butyl. allyl and the like groups.

### Effects

The inventors reported that ascofuranone orally administered to rodents demonstrates the effect of lowering serum lipid in rats bred with either a standard diet or a high-fat diet with very low toxicity (J. Antibiotics 24: 681-686, 1973 and Jpn. J. Pharmacol. 25: 35-39, 1975). The inventors also reported that ascofuranone orally administered to DOCA hypertension rats as a hypertension model does not suppress pressure rise but improves indications of cardiovascular diseases (Eur. J. Pharmacol. 69: 429-438, 1981). The reason why ascofuranone demonstrates such effect has long been a riddle. It was very recently found that ascofuranone has such effect by functioning as a ligand for PPAR belonging to the nuclear receptor superfamily to regulate the expression of genetic information.

In the prior application, the inventors found that ascochlorin and its analogous compounds and derivatives thereof dose-dependently increase the expression of RXR or RAR-binding site-coupled transfer genes in cell culture and demonstrated that they are ligands for RXR and RAR receptors. The applicants filed a patent application for a medical use based on these findings.

Among compounds of the present invention, ascofuranone and its analogous compounds and derivatives thereof are specific ligands for PPAR receptors and cannot activate retinoid X (RXR) and ol-trans-retinoic acid (RAR) receptors. A group of compounds obtained by modifying the hydroxyl group at the 2-position and/or 4-position of the orcylaldehyde of ascochlorin with a substituent activate both PPAR and RXR belonging to the nuclear receptor superfamily to enhance the expression of transfer genes. Accordingly, they have a major characteristic in that they are ligands binding two receptors. Thus, compounds of the present invention have the almost same pharmacological effects as those of compounds of the prior application.

Pharmacological effects shown by compounds of the present invention are as follows. First, they have the effect of alleviating life style-related diseases such as type II diabetes, essential hypertension, ischemic heart diseases, hyperlipidemia and complications thereof. Compounds of the present invention are also effective to treat and prevent malignant tumor suffering high expression of PPAR and conditions associated therewith. Specifically, they can be effectively used to treat terminal gastrointestinal cancer suffering high expression of PPAR and cachexia associated therewith. Compounds of the present invention are also effective to treat and prevent chronic inflammation in various organs and tissues. Typical diseases to which they can be applied include chronic vascular inflammation associated with periarteritis nodosa (aneurysms), coronary arteriosclerosis, cerebral angiopathy, microangiopathy of diabetes, chronic rheumatoid arthritis, etc. Compounds of the present invention have a major characteristic in that they are not specific ligands activating PPARγ only expressed in white adipose tissue like TZDs but also activate PPARγ widely expressed in lymphatic tissue, adrenal gland and intestines. As PPARs are expressed in vascular smooth muscle cells, transcriptional control of inflammatory cytokine genes by activation of PPARs can be expected to decrease inflammatory cytokine transcriptional factor NF-κB, thereby improving chronic vascular inflammation (B. Staels et al., Nature, 393: 790-795, 1998). Thus, compounds of the present invention are effective in the treatment of various diseases caused by overproduction of adipocytokines and inflammatory cytokines without being limited to the application range specifically described herein. The reason why compounds of the present invention show a wide spectrum of pharmacological effects compared with thiazolidinedione compounds (TZD compounds) that also activate nuclear receptor PPARs seems to lie in the difference of binding manner to receptors. That is, TZD compounds never bind a PPARγ receptor-immobilized BIAcore column, but compounds of the present invention bind PPARγ receptor even in vitro.

### Formulations

Compounds of the present invention can be administered in a pure form or in the form of an appropriate pharmaceutical composition via any route acceptable for drugs used for similar applications. Thus, they can be administered, for example, orally, intranasally, parenterally or topically in the form of a solid, semisolid, freeze-dried powder or liquid such as tablets, suppositories, pills, capsules, powders, solutions, suspensions, emulsions, creams, lotions, aerosols, ointments and gels, preferably in an appropriate unit dosage form that allows an exact dose to be administered. This composition comprises an ordinary pharmaceutical carrier or excipient and a compound of the present invention optionally with other pharmaceutical products, carriers and absorption aids. Generally, pharmaceutically acceptable compositions contain about 1-99% (by weight) of a compound of the present invention and about 99-1% of appropriate pharmaceutical additives, depending on the dosage form to be administered. The compositions contain about 5-75% of a compound of the present invention as a pharmaceutical product with the remainder being appropriate pharmaceutical excipients. The effective daily dose of compounds of the present invention to improve pathology is 0.1-20 mg/kg of adult body weight, desirably 0.2-5 mg/kg.

Preferred dosage forms for diseases specifically described above should be formulated in such a manner that a variable dose can be applied depending on the severity of the disease. What is most important for formulation is the limitation from the fat solubility of compounds of the present invention. Ligands for the nuclear receptor superfamily are fat-soluble hormones or vitamins, and therefore, compounds of the present invention are also fat-soluble as a matter of course. Pharmaceutically acceptable additives for oral administration include any normally suitable excipients such as mannitol, lactose, starch, magnesium stearate, sodium saccharin, talc, cellulose, glucose, gelatin, sucrose and magnesium carbonate. Such compositions can be in the form of solutions, tablets, pills, capsules, powders and sustained release formulations. The compositions are preferably in the form of a tablet or pill containing a compound of the present invention in combination with diluents such as lactose, sucrose and dicalcium phosphate; disintegrating agents such as starch and derivatives thereof; lubricants such as magnesium stearate; binders such as starch, gum arabic, polyvinylpyrrolidone, gelatin, cellulose and derivatives thereof; surfactants for wetting the surfaces of highly fat-soluble and water-repellent particles of the compound of the present invention; fat-soluble additives, bile acid, phospholipids, etc. It is especially preferred that synthetic aliphatic surfactants or polymer additives soluble in organic solvents are contained. Examples thereof include gum arabic, sodium alginate, methylcellulose, carboxymethylcellulose, hydroxypropylcellulose, polyvinylpyrrolidone, bentonite, sodium laurylsulfate, Polysorbate 80, sorbitan monofatty acid esters, polyoxyl 40 stearate, etc.

What is especially important for formulation is the limiting of the water-repellent physical properties of compounds of the present invention. Generally, gastrointestinal absorption of fat-soluble drugs is governed by the speed at which each molecule is dissolved from particles of the drugs in water. This is because fat-soluble drugs dissolved in water are rapidly absorbed virtually without barrier once they reach absorption sites of gastrointestinal mucosa. Even less water-soluble drugs are more rapidly absorbed to increase bioavailability if they are rapidly dissolved in water. However, compounds of the present invention are known to dissolvevery slowly in water. Therefore, they must be formulated by selecting additives and formulation processes to increase the dissolution speed in water. One means for improving dissolution in water is to decrease the diameter of drug particles as far as possible, ie, (1) to form fine particles in order to increase the surface areas of particles in contact with water. However, even fine particles cannot come into contact with water because of the water repellency of the particle surfaces and the dissolution speed in water cannot be increased unless they are dispersed. Therefore, it is necessary (2) to combine compounds of the present invention with a surfactant or a polymer having both fat-soluble and water-soluble groups in order for particle surfaces to come into contact with water. It is also essential (3) to make it easy for each molecule to be released from drug particles. That is, in the case of crystalline drug particles, it is necessary to select such a crystal form that requires little free energy to allow each molecule to be released from the crystal lattice and also to include a formulation process for amorphizing compounds of the present invention to make it easy for each molecule to be released. All these conditions necessary for formulating compounds of the present invention are as defined in the claims for compounds of the present invention.

### Schiff base

Compounds of the present invention can form a Schiff base by the reaction between the aldehyde group and an amino group of a serum protein in blood. The Schiff base formed by compounds of the present invention with a serum protein has a buffer effect by temporarily masking biological activity of educts absorbed from digestive tracts and gradually supplying the active educts to a target tissue. This Schiff base is adsorbed to the surfaces of target cells and taken into the cells via endocytosis, where it is hydrolyzed to regenerate a free compound. The free compound is dissolved in oil drops in stored fat and dissolved little by little in the cytoplasm in fat cells to produce its effect. Further, it is transported with stored fat of white adipose tissue when the fat is transported as an energy source to other tissues. Thus, it also has the role of storing.

Compounds of the present invention are hardly taken into other tissues, and are neither accumulative nor toxic because of the absence of oil droplets of stored fat.

The following examples further illustrate the present invention without, however, limiting the same thereto as a matter of course.

### Preparation 1

### Dehydroascofuranone

Dehydroascofuranone obtained by removing two hydrogen molecules from the tetrahydrofuran ring of ascofuranone was synthesized as follows. To a solution of 0.16 g (4.0 mmol) of sodium hydroxide dissolved in 0.7 ml of deionized water was added by portions a solution of AgNO₃ (0.34 g, 2.00 mmol) in deionized water (0.7 ml) with cooling in water bath. To the resulting aqueous suspension of silver nitrate was added a solution of ascofuranone (0.2 g, 0.476 mmol) in dioxane (1.4 ml). The reaction mixture was stirred at room temperature for 3 hours and then diluted with a mixed solvent of water/dioxane (1:1) and filtered through celite, and the filtrate was acidified with 6 N HCl and then extracted with dichloromethane. The dichloromethane layer was washed with water and saturated saline and then dried over MgSO₄ and concentrated under reduced pressure. The residue was purified by silica gel II column chromatography with dichloromethane/acetone (60:1) to give 0.047 g of dehydroascofuranone (yield 24%).

mp 139.5-140.5°C. IR (cm⁻¹): 1680, 1635, 1555. ¹H NMR (ppm): 1.35 (6H, s), 1.82 (3H, br s), 1.84 (3H, br s), 2.13 (2H, br t, J=7.2 Hz), 2.33 (2H, br q, J=7.5 Hz), 2.60 (3H, s), 3.40 (2H, d, J=7.2 Hz). 10.14 (1H, s), 12.69 (1H, s). ¹³C NMR (ppm): 12.93, 14.44, 16.13, 22.03, 23.03 (probably two overlapping peaks), 27.05, 38.29, 88.10, 98.52, 113.14, 113.59, 114.21, 121.90, 125.76, 135.03, 137.71, 138.12, 156.17, 162.15, 184.66, 193.30, 207.20. Elementary analysis :Found (%): C, 65.88; H, 6.54. Calculated (%): C, 65.94; H, 6.50.

### Preparation 2

### A. 4-O-Ethoxycarbonylmethyl-2-O-methylascochlorin (1): compound 27

Sodium hydroxide (60%, 23.1 mg, 0.578 mmol) was washed with pentane and then suspended in DMF (1 ml). To this suspension was added dropwise a solution of 2-O-methylascochlorin (0.220 g, 0.525 mmol) in DMF (2 ml), and the mixed solution was stirred at room temperature for 30 minutes and then stirred with ethyl bromoacetate (0.0641 ml, 0.578 mmol) at 50°C for 4 hours. The reaction solution was taken in saturated aqueous ammonium chloride solution and extracted with ether, and the organic layer was washed successively with water and saturated saline, and then dried over anhydrous magnesium sulfate. After the desiccant was filtered off, the filtrate was concentrated under reduced pressure and the residue was purified by silica gel column chromatography (15 g SiO₂, hexane/ethyl acetate) to give 0.204 g (77%) of **(1)** (as a colorless gum).

IR (film): 1765, 1713, 1694. NMR (CDCl₃, 500 MHz): 0.70 (3H, s), 0.80 (3H, d, J=6.7 Hz), 0.83 (3H, d, J=6.9 Hz), 1.31 (3H, t, J=7.0 Hz), 1.58-1.67 (1H, m), 1.90 (3H, br s), 1.90-1.96 (2H, m), 2.34-2.44 (3H, m), 2.63 (3H, s), 3.65 (2H, d, J=7.0 Hz), 3.88 (3H, s), 4.29 (2H, q, J=7.0 Hz), 4.61 (2H, s), 5.39 (1H, d, J=16.0 Hz), 5.46 (1H, t, J=7.0 Hz), 5.89 (1H, d, J=16.0 Hz), 10.42 (1H, s). ESMS (electrospray mass spectrum, positive) (m/z): 505 {(M+H)⁺, C₂₈H₃₇O₆³⁵Cl + H⁺}, 507 {(M+H)⁺, C₂₈H₃₇O₆³⁷Cl + H⁺}.

### B. 4-O-Carboxymethyl-2-O-methylascochlorin (2). compound 27

Compound (1) obtained above (0.207 g, 0.409 mmol) was dissolved in methanol (6.2 ml) and stirred with 20% aqueous potassium carbonate solution (1 ml, 1.45 mmol) at room temperature for 2 hours. The reaction solution was adjusted to pH 2 with 3 N hydrochloric acid and then extracted with ether. The organic layer was washed successively with water and saturated saline, and then dried over anhydrous magnesium sulfate. After the desiccant was filtered off, the filtrate was concentrated under reduced pressure and the residue was purified by silica gel column chromatography (15 g SiO₂, dichloromethane/ethanol) to give 0.122 g (63%) of **(2)** (as a colorless gum).

IR (film): 1715, 1696. NMR (CDCl₃, 500 MHz): 0.70 (3H, s), 0.81 (3H, d, J=6.8 Hz), 0.83 (3H, d, J=6.8 Hz), 1.62 (1H, qd, J=13.5, 6.0 Hz), 1.91 (3H, br s), 1.91-1.97 (2H, m), 2.34-2.45 (3H, m), 2.63 (3H, s), 3.62 (2H, d, J=6.9 Hz), 3.84 (3H, s), 4.65 (2H, s), 5.42 (1H, d, J=16.0 Hz), 5.43 (1H, t, J=6.9 Hz), 5.89 (1H, d, J=16.0 Hz), 10.42 (1H, s).

### Preparation 3

### 4-O-(4-Carboxybutanoyl)ascochlorin (4); compound 29

To a solution of ascochlorin (0.150 g, 0.358 mmol) in anhydrous pyridine (1.5 ml) were added 4-dimethylaminopyridine (4.4 mg, 0.036 mmol) and glutaric anhydride (0.049 g, 0.430 mmol). The reaction solution was stirred at 50°C overnight and then adjusted to pH 2 with 1 N hydrochloric acid and extracted with ethyl acetate. The organic layer was washed with saturated saline, and then dried over anhydrous magnesium sulfate. After the desiccant was filtered off, the filtrate was concentrated under reduced pressure and the residue was purified by silica gel column chromatography (13 g SiO₂, hexane/ethyl acetate) to give 0.081 g (42%) of **(4)** as a colorless crystal.

mp 120-123°C. IR(KBr disc): ∼3000, 1771, 1711, 1649. NMR (CDCl₃, 500 MHz): 0.70 (3H, s), 0.81 (3H, d, J=7.0 Hz), 0.83 (3H, d, J=7.0 Hz), 1.59-1.66 (1H, m), 1.88 (3H, s), 1.89-1.96 (2H, m), 2.12 (2H, qui, J=7.2 Hz), 2.34-2.45 (3H, m), 2.55 (2H, t, J= 7.2 Hz), 2.65 (3H, s), 2.76 (2H, t, J=7.2 Hz), 3.42 (2H, br d, J=6.0 Hz), 5.35 (1H, t, J=7.0 Hz), 5.39 (1H, d, J=16.0 Hz), 5.87 (1H, d, J=16.0 Hz), 10.30 (1H, s), 12.54 (1H, s).

### Preparation 4

### 4-O-Isonicotinoyl-2-O-methylascochlorin (5): compound 31

To a solution of 2-*O*-methylascochlorin (61.3 mg, 0.146 mmol) in anhydrous pyridine (1 ml) was added isonicotinoyl chloride hydrochloride (71.7 mg, 0.403 mmol). The reaction solution was stirred at room temperature for 3 hours, and then further stirred with water for 30 minutes and extracted with ethyl acetate. The organic layer was washed successively with saturated aqueous copper sulfate solution, water, saturated aqueous sodium bicarbonate solution and saturated saline, and then dried over anhydrous magnesium sulfate. After the desiccant was filtered off, the filtrate was concentrated under reduced pressure and the residue was purified by silica gel column chromatography (10 g SiO₂, hexane/ethyl acetate) to give 69.3 mg (90%) of **(5)** (as a colorless gum).

IR(film): 1760, 1710, 1700. NMR (CDCl₃, 500 MHz): 0.69 (3H, s), 0.76-0.83 (6H, br), 1.59-1.64 (1H, m), 1.65 (3H, br s), 1.85-1.96 (2H, m), 2.32-2.45 (3H, m), 2.67 (3H, s), 3.39-3.50 (1H, br), 3.50-3.63 (1H, br), 3.88 (3H, s), 5.29 (1H, d, J=16.0 Hz), 5.34 (1H, t, J=7.0 Hz), 5.82 (1H, d, J=16.0 Hz), 7.79-7.99 (2H, m), 8.87 (2H, m), 10.49 (1H, s). ESMS (electrospray mass spectrum, positive) (m/z): 524 {(M+H)⁺, C₃₀H₃₄NO₅³⁵Cl + H⁺}, 526 {(M+H)⁺, C₃₀H₃₄NO₅³⁷Cl + H⁺}.

### Preparation 5

### 4-O-Isonicotinoylascochlorin (6): compound 30

To a solution of ascochlorin (0.200 g, 0.494 mmol) in anhydrous pyridine (1 ml) was added isonicotinoyl chloride hydrochloride (0.242 g, 1.36 mmol). The reaction solution was stirred at room temperature for 3 hours, and then further stirred with water for 30 minutes and extracted with ether. The organic layer was washed successively with saturated aqueous copper sulfate solution, water, saturated aqueous sodium bicarbonate solution and saturated saline, and then dried over anhydrous magnesium sulfate. After the desiccant was filtered off, the filtrate was concentrated under reduced pressure and the resulting crystal was purified by recrystallization from hexane/ether to give 0.199 g (79%) of **(6)** as a colorless crystal.

mp 119-121°C. IR(film): 1752, 1711, 1642. NMR (CDCl₃, 500 MHz): 0.68 (3H, s), 0.79 (3H, d, J=7.0 Hz), 0.81 (3H, d, J=7.0 Hz), 1.59-1.65 (1H, m), 1.68 (3H, br s), 1.86-1.96 (2H, m), 2.33-2.44 (3H, m), 2.69 (3H, s), 3.34-3.46 (1H, br), 3.46-3.60 (1H, br), 5.29 (1H, d, J=16.0 Hz), 5.36 (1H, t, J=7.0 Hz), 5.82 (1H, d, J=16.0 Hz), 8.01 (2H, br d, J=4.6 Hz), 8.85-8.95 (2H, br), 10.35 (1H, s), 12.60 (1H, s). ESMS (electrospray mass spectrum, negative) (m/z): 508 {(M-H)⁻, C₂₉H₃₂NO₅³⁵Cl - H⁺}, 510 {(M-H)⁻, C₂₉H₃₂NO₅³⁷Cl - H⁺}.

### Preparation 6

### A. 4-O-Acetyl-2-O-methvascofuranone (8); precursor of compound 27

To a solution of 4-O-ascofuranone (0.301 g, 0.651 mmol) in acetone (2 ml) were added potassium carbonate (99 mg, 0.716 mmol) and methyl iodide (0.0484 ml, 0.781 mmol), and the mixed solution was heated under reflux for 2 hours. The reaction solution was filtered and then the filtrate was diluted with ether and washed successively with water, saturated aqueous sodium bicarbonate solution and saturated saline, and then dried over anhydrous magnesium sulfate. After the desiccant was filtered off, the filtrate was concentrated under reduced pressure and the residue was purified by silica gel column chromatography (10 g SiO₂, hexane/ethyl acetate) to give 75.5 mg (24%) of **(8)** (as a colorless gum).

NMR (CDCl₃, 500 MHz): 1.22 (3H, s), 1.28 (3H, s), 1.64 (3H, s), 1.77 (3H, br s), 2.04 (2H, t, J=7.8 Hz), 2.09-2.20 (2H, m), 2.35 (3H, s), 2.39 (1H, dd, J=18.2, 10.0 Hz), 2.47 (1H, dd, J=18.0, 6.2 Hz), 2.63 (3H, s), 3.33 (2H, d, J=6.5 Hz), 3.83 (3H, s), 4.52 (1H, dd, J=10.0, 6.2 Hz), 5.08 (1H, tq, J=6.5, 1.3 Hz), 5.52 (1H, t, J=6.6 Hz), 10.44 (1H, s).

### B. 2-O-methylascofuranone (7); compound 27

Compound **(8)** obtained above (63.5 mg, 0.133 mmol) was dissolved in methanol (4.2 ml) and stirred with 1% aqueous sodium hydroxide solution (4.2 ml, 1.05 mmol) at room temperature for 4 hours. After the reaction solution was neutralized with 2 N hydrochloric acid, methanol was distilled off and the residue was diluted with water and extracted with ethyl acetate. The organic layer was washed successively with water, saturated aqueous sodium bicarbonate solution and saturated saline, and dried over anhydrous magnesium sulfate. After the desiccant was filtered off, the filtrate was concentrated under reduced pressure and the residue was purified by silica gel column chromatography (10 g SiO₂, hexane/ethyl acetate) to give 52.1 mg (90%) of **(7)** (as a colorless gum).

IR(film): 3390, 1756, 1686, 1564. NMR (CDCl₃, 500 MHz): 1.22 (3H, s), 1.29 (3H, s), 1.64 (3H, br s), 1.80 (3H, br s), 2.06 (2H, t, J=7.5 Hz), 2.12-2.22 (2H, m), 2.40 (1H, dd, J=18.0, 10.0 Hz), 2.47 (1H, dd, J=18.0, 6.5 Hz), 2.66 (3H, s), 3.42 (2H, d, J=6.6 Hz), 3.82 (3H, s), 4.53 (1H, dd, J=10.0, 6.5 Hz), 5.20 (1H, tq, J=6.9, 1.2 Hz), 5.51 (1H, t, J=6.9 Hz), 6.35 (1H, s), 10.37 (1H, s). ESMS (electrospray mass spectrum, negative) (m/z): 433 {(M-H)⁻, C₂₄H₃₁O₅³⁵Cl - H⁺}, 435 {(M-H)⁻, C₂₄H₃₁O₅³⁷Cl - H⁺}.

### Preparation 7

### 4-O-(4-Carboxybutanoyl)ascofuranone (9); compound 15

To a solution of ascofuranone (0.100 g, 0.238 mmol) in anhydrous pyridine (1 ml) were added 4-dimethylaminopyridine (2.9 mg, 0.04 mmol) and glutaric anhydride (0.033 g, 0.490 mmol). The reaction solution was stirred at 50°C overnight and then adjusted to pH 2 with 1 N hydrochloric acid and extracted with ethyl acetate. The organic layer was washed with saturated saline, and then dried over anhydrous magnesium sulfate. After the desiccant was filtered off, the filtrate was concentrated under reduced pressure and the residue was purified by silica gel column chromatography (10 g SiO₂, hexane/ethyl acetate) to give 0.040 g (32%) of **(9)** (as a colorless gum).

IR(film): -3000, 1752, 1713, 1638. NMR (CDCl₃, 500 MHz): 1.23 (3H, s), 1.29 (3H, s), 1.63 (3H, br s), 1.74 (3H, br s), 2.02 (2H, t, J=7.5 Hz), 2.11 (2H, qui, J=7.0 Hz), 2.12-2.17 (2H, m), 2.37 (1H, dd, J=18.2, 10.0 Hz), 2.44 (1H, dd, J=18.2, 6.3 Hz), 2.54 (2H, t, J=7.0 Hz), 2.65 (3H, s), 2.76 (2H, t, J=7.0 Hz), 3.24-3.31 (2H, br), 4.55 (1H, dd, J=10.0, 6.3 Hz), 5.07 (1H, tq, J=7.0, 1.5 Hz), 5.50 (1H, br t, J=7.0 Hz), 10.30 (1H, s), 12.52 (1H, s).

### Preparation 9

### 4-O-Isonicotinoylascochlorin (10): compound 14

To a solution of ascofuranone (0.200 g, 0.476 mmol) in anhydrous pyridine (1 ml) was added isonicotinoyl chloride hydrochloride (0.233 g, 1.31 mmol). The reaction solution was stirred at room temperature for 1 hour, and then further stirred with water for 30 minutes and extracted with ether. The organic layer was washed successively with saturated aqueous copper sulfate solution, water, saturated aqueous sodium bicarbonate solution and saturated saline, and then dried over anhydrous magnesium sulfate. After the desiccant was filtered off, the filtrate was concentrated under reduced pressure and the residue was purified by silica gel column chromatography (10 g SiO₂, hexane/ethyl acetate) to give 0.135 g (54%) of **(10)** (as a colorless gum).

IR(film): 1756, 1644. NMR (CDCl₃, 500 MHz): 1.21 (3H, s), 1.28 (3H, s), 1.53 (3H, br s), 1.62 (3H, br s), 1.88-1.97 (2H, m), 2.02-2.12 (2H, m), 2.35 (1H, dd, J=18.0, 10.0 Hz), 2.42 (1H, dd, J=18.0, 6.3 Hz), 2.69 (3H, s), 3.21-3.33 (1H, br), 3.33-3.46 (1H, br), 4.51 (1H, dd, J=10.0, 6.3 Hz), 5.09 (1H, br t, J=7.0 Hz), 5.48 (1H, br t, J=7.0 Hz), 8.00-8.02 (2H, m), 8.89-8.91 (2H, m), 10.35 (1H, s), 12.60 (1H, s). ESMS (electrospray mass spectrum, negative) (m/z): 524 {(M-H)⁻, C₂₉H₃₂NO₆³⁵Cl - H⁺}, 526 {(M-H)⁻, C₂₉H₃₂NO₆³⁷Cl - H⁺}.

### Example 1

Various materials were mixed and granulated by a standard procedure and compacted into tablets each containing 63 mg of compound 1, 10 mg of sorbitan sesquioleate, 10 mg of powdered silica gel, 40 mg of corn starch 50 mg of L-hydroxypropylcellulose (PO-30), 12 mg of hydroxypropylcellulose-SL, 46 mg of microcrystalline cellulose, 10 mg of carboxymethylcellulose sodium salt, 3 mg of calcium stearate and 6 mg of talc.

### Example 2

A mixture of 63 g of ground compound 11, 50 g of lactose, 60 g of corn starch, 30 g of L-hydroxypropylcellulose, 12 g of hydroxypropylcellulose-SL, 46 g of microcrystalline cellulose, 10 g of carboxymethylcellulose II sodium salt, 3 g of calcium stearate and 6 g of talc was granulated by a standard procedure and compacted into tablets each weighing 0.25 g.

### Example 3

A mixture of 100 g of compound 21, 70 g of microcrystalline cellulose, 35 g of lactose, 70 g of carboxymethylcellulose calcium salt, 70 g of corn starch and 5 g of magnesium stearate was granulated by a standard procedure and compacted into tablets each weighing 0.35 g.

### Example 4

In a transfer gene activity assay system, compounds of the present invention enhanced the expression of a reporter gene. Thus, COS-1 cells transfected with a plasmid bearing a reporter gene whose expression is controlled by PPAR-responsive elements and a PPARγ expression plasmid show increased expression of the reporter gene when they were treated with compounds of the present invention. This result shows that compounds of the present invention are agonists for PPARs and that these compounds control gene expression via PPARs.

### Transfection of plasmids into COS-1 cells:

Transfection was performed by a standard procedure using a LipofectAMINE reagent from Life Technologies. A 35 mm Petri dish containing 1 x 10⁵ cells dispersed in 2 ml of Dulbecco's minimum medium (DMEM) containing 10% fetal bovine serum was incubated in a CO₂ incubator for 17 hours. Then, the medium was removed and cells adhered to the Petri dish were washed with phosphate buffered physiological saline (PBS), after which transfection was performed using a LipofectAMINE reagent. That is, 500 ml of a minimum serum medium OptiMEM (Life Technologies) containing 4 mg of a plasmid bearing the luciferase gene whose expression is controlled by PPAR-responsive elements, 4 mg of a PPAR expression plasmid and 2 mg of a plasmid bearing the β-galactosidase gene for monitoring transfection efficiency was preliminarily mixed with 500 ml of OptiMEM containing 50 ml of LipofectAMINE dissolved therein, and the mixed solution was maintained at room temperature for 15 minutes. A Petri dish containing a mixture of 100 ml of this mixed solution and 400 ml of OptiMEM was incubated in a CO₂ incubator at 37°C for 3 hours. The transfection medium was aspirated off from the Petri dish, which was supplied with DMEM containing 10% serum treated with activated carbon and a test compound and incubated at 37°C for 20 hours. Test compounds were compounds 1, 11, 12 and 20, and controls were pioglitazone and troglitazone. The medium also contains a solvent for test compounds dimethyl sulfoxide (final concentration 0.1%).

After the medium in the Petri dish was aspirated off, 250 ml of a lysing solution for cultured cells (25 mM Tris phosphate buffer (pH 7.8), 2 mM 1,2-diaminocyclo-hexane-N, N,N',N'-tetraacetate, 10% glycerol, 1% Triton X-100) was added and cells were scraped with rubber policeman. Cells were completely disrupted by freezing and thawing and then centrifuged at 4°C (15,000 rpm, 2 min), and the supernatants were used as test samples. To 20 ml of the supernatants was added 100 ml of a luciferase substrate solution (2.14 mg Co-A, 100 ml 470 mM D-Luciferin, 200 ml 530 mM ATP, 2 ml DTT-Tricine buffer (pH 7.8)) and the mixed solutions were measured for fluorescence in a luminometer (ATTO luminosensor AB-2000). The β-galactosidase for monitoring transfection efficiency was measured for fluorescence in a luminometer using Luminescent β-galactosidase detection kit II (Clontech). The results are shown in Table 5, which demonstrates that compounds of the present invention are specific ligands for PPARs.

**Table 5.**

| Compounds of the present invention activate PPAR receptors and enhance transcription of a transfer gene | | | |
|---|---|---|---|
| Compounds | Activation of receptors | | |
| | PPARα | PPARβ | PPARγ |
| Troglitazone | - | - | ++++ |
| Pioglitazone | - | - | ++++ |
| Compound 1 | - | - | +++ |
| Compound 7 | - | - | +++ |
| Compound 21 | - | - | ++ |
| Compound 25 | - | - | +++ |
| Compound 26 | - | - | ++ |
| Compound 27 | - | - | ++ |
| Compound 29 | - | - | +++ |
| Dehydroascofuranone | ++ | - | - |
| The extent of activation was evaluated as follows: ++++: Activation peak appears at <10⁻⁶M. +++: Activation peak appears at 10⁻⁶-10⁻⁵M. ++: Activation peak appears at 10⁻⁵-10⁻⁴M. +: Activation peak appears at >10⁻⁴M. | | | |

### Example 5

Compounds of the present invention show the effect of preventing periarteritis nodosa and aneurysms. In order to evaluate the effect of preventing aneurysms, a quantitatively assayable rat hypertension model was used so that aneurysms formed in mesenteric arteries were measured and statistically processed to determine the effect. The membrane was removed with forceps from isolated mesenterium and the remaining arterioles were fixed with formalin and then fat-stained. The number of arterioles and the number of aneurysms were counted in triplicate to calculate the average number of aneurysms per arteriole. As a hypertension model, rats with one kidney extracted to decrease renal function were given 0.9% saline as drinking water having a slightly higher osmosis than plasma and subcutaneously received 10 mg/kg of a mineral corticoid deoxycorticosteron acetate (DOCA) weekly. The left kidney was extracted from male Wistar rats (12 animals) under anesthetization. The rats deprived of kidney were randomly divided into two groups (n = 6) after a period of recovery for a week. One of the two groups was a control group and the other received compound 1. Compound 1 was compulsorily orally administered at 100 mg/kg from week 3 after the start of experiments. The experimental period was 7 weeks, during which feed intake, drinking water intake, urine volume, body weight and blood pressure were measured every 3 days from the start of experiments.

Blood pressure rapidly rose and the average blood pressure exceeded 180 mmHg at week 3. In the group that orally received compound 1 from week 3, any significant effect of suppressing pressure rise was not observed as compared with the control group. At week 7, the animals were dissected and mesenteric arteries were isolated and treated with formalin and then fat-stained to count the number of aneurysms. In the hypertension control group, all the individuals showed aneurysms with the average being 1.72 ± 0.74 aneurysms (mean ± SE) per mesenteric artery, suggesting that hypertensive arterial lesion advanced. The group treated with compound 1 had an average of 0.51 ± 0.37 aneurysms (mean ± SE) per mesenteric artery. Compounds of the present invention clearly suppress the advance from periarteritis nodosa to aneurysms.

### Example 6

The effect of compound 17 that improves polydipsia/polyuria and inhibits urinary sugar excretion in hereditary obese and diabetic mice C57BL/ksj db/db was demonstrated by the experiments below. Ten male db/db mice at 30-32 weeks of age were randomly divided into two groups (n = 5), ie, a control group and compound 17 group. The animals were allowed free access to diet and drinking water. The diet was mouse II rat standard food CE-2 powder from Clea Japan, which was pelletized after a solution of compound 17 in acetone was applied and acetone was evaporated by air-drying. Compound 17 was added in an amount of 0.1% of the diet. A control diet was also pelletized after the same amount of acetone was applied.

Both groups received the control diet for first 7 days to test feed intake, urine volume, urinary sugar level and urinary sugar excretion level. During the following 5 days, the treatment group received the diet containing 0.1% compound 17. The treatment group was bred with the control diet again for further 4 days and then bred with the diet containing 0.1% compound 17 again for 5 days on days 20-24. As db/db mice individually bred in metabolic cages show wide variation in feed and drinking water intakes, 5 animals were housed in a rat metabolic cage and the values obtained in each cage were divided by 5 and shown in Fig. 1. As shown in Fig. 1, urine volume, urinary sugar level and urinary sugar excretion level sharply decrease when the diet containing compound 17 is given. Urinary sugar excretion level is 50% or less as compared with the control period even 24 hours after administration is stopped, showing that compound 17 has a carry-over effect.

### Example 7 (Ascofuranone has the effect of relieving streptozotocin-induced diabetes)

Streptozotocin (50 mg/kg) was injected into the tail vein of male Wistar rats weighing about 250 g to prepare an insulin deficiency diabetes model. Compound 1 suspended in a gum arabic solution was orally administered at 100 mg/kg once daily, starting immediately after administration of streptozotocin. The animals were housed in a urine-collecting cage for 24 hours on days 7 and 14 to collect urine for 24 hours. As shown in Table 6, drinking water intake of the diabetes control group increased 4.1-fold on day 7 and 4.5-fold on day 14 as compared with that of the healthy control group. In contrast, the intake was kept at a significantly low level such as 41.4% on day 7 and 43.6% on day 14 in the group treated with compound 1 as compared with the diabetes control group. The table shows feed intake, drinking water intake, urine volume and urinary sugar excretion level in the 3 groups. Feed intake increased about 1.5-fold in the diabetes control group as compared with the healthy control group, and significantly decreased 14.3% on day 7 and 23.3% on day 14 in the group treated with compound 1 as compared with the diabetes control group. However, weight gain was 80 g/week in the healthy group in contrast with only 18 g/week in the diabetes control group. The group treated with compound 1 showed a weight gain of 30 g/week despite the lower feed intake than in the diabetes control group. This means that administration of compound 1 improved metabolism to greatly reduce urinary sugar excretion.

**Table 6**

| | Day | Diabetes control group | Diabetes-compound 1 group | Healthy control group |
|---|---|---|---|---|
| Feed intake (g/rat) | 7 | 38.4±1.7 | 32.9±1.9* | 27.0±3.2* |
| | 14 | 42.5±1.7 | 32.6±2.9* | 26.8±0.9** |
| Drinking water intake (ml/rat/day) | 7 | 169.5±7.6 | 99.9±13.0** | 41.6±1.1** |
| | 14 | 189.6±5.7 | 106.9±18.0** | 42.4±1.0** |
| Urine volume (ml/rat/day) | 7 | 128.5±5.3 | 74.6±6.4** | 19.8±1.7** |
| | 14 | 159.8±5.3 | 79.7±12.5** | 17.5±0.8** |
| Urinary sugar excretion (g/rat/day) | 7 | 8.85±0.36 | 5.39±0.55** | N.D. |
| | 14 | 12.05±0.38 | 5.78±1.09** | N.D. |
| Body weight (g/rat) | 7 | 291.0±4.7 | 307.7±4.8 | 350.4±10.2** |
| | 14 | 292.5±4.2 | 318.3±6.8* | 370.4±10.2** |

| | | | | |
|---|---|---|---|---|
| Diabetes control group, n=9; Diabetes-compound 1 group, n=8; healthy control group, n=5. *P<0.05, | | | | |
| **P<0.01, in Student's t-test. | | | | |

### Example 8

Blood was collected from the tail vein of ten db/db mice at 12 weeks of age, and the animals were divided into two groups, ie, a control group and a compound 25 group, each having an average blood glucose level of about 700 mg/dl and housed in urine-collecting cages. A small amount of blood was collected from the tail vein on days 1, 2, 3, 5, 7 and 10 from the start of experiments to determine blood glucose. Urine was collected for 24 hours at the same intervals to determine urinary sugar excretion level by the oxygen method. The results are shown in Fig. 2, which demonstrates that urinary sugar decreased to a half on the day following the start of administration and that excretion level loss was over 90% on and after day 3. Blood glucose significantly decreased from day 3 of administration and its loss was over 30% on day 7.

### Example 9

Forty female NOD mice weighing 25 g were divided into 4 groups, ie, a control group, a group treated with compound 1 (50 mg/kg), a group treated with compound 1 (25 mg/kg) and a group treated with compound 25 (50 mg/kg). Compound 1 and compound 25 were suspended in a 2% gum arabic solution and intraperitoneally administered twice weekly on Monday and Friday. The mice were bred for 24 weeks, during which they were tested for urinary sugar excretion using an urinary sugar detection tape on Monday and Friday (Fig. 3).

### Example 10

A Surface Plasmon Resonance sensor (BIAcore column) was used to examine whether or not compounds of the present invention bind immobilized PPARγ receptor. Fig. 4 shows sensorgrams of compound 27 and pioglitazone used as a control. The figure shows that compounds of the present invention bind PPARγ receptor even in vitro, but the control pioglitazone does not bind it.

### Example 11 (Compound 1 prevents hyperlipidemia in tumor bearing mice)

In these experiments, serum neutral fat was selected as an indication of cachexia to serve as an auxiliary marker for knowing dynamics of TNFα that is difficult to quantitize, because it is difficult to exactly measure blood TNFα and it is easier to measure serum neutral fat than blood TNFα. Forty male ddY-strain mice at 5 weeks of age were randomly divided into 5 groups (n = 8), among which 4 groups were intraperitoneally implanted with 2 x 10⁶ Ehrlich ascites tumor cells. The group implanted with no tumor cells was a healthy control group. From 24 hours after implantation of tumor cells, 3 groups intraperitoneally received 100-400 mg/kg of compound 1, while the other group received a solvent 0.1% Tween 80 (0.2 ml) alone free from compound 1. On day 8 after implantation of tumor cells, blood was collected from the heart of tumor bearing mice to assay neutral fat in separated serum and detect blood TNFα by immunoelectrophoresis.

**Table 7.**

| Compound 1 has the effect of preventing high blood neutral fat in tumor bearing mice (mean±SD) | | | | |
|---|---|---|---|---|
| Dose (mg/kg) | Body weight (g/mouse) | Serum neutral Serum neutral fat (mg/dl) | Statistic significant difference^{a)} | Blood TNFα |
| 0 | 40.0±1.0 | 401.1±73.8 | | ++++ |
| 100 | 38.0±1.0 | 258.2±53.2 | NS | +++ |
| 200 | 36.7±0.7 | 185.1±26.2* | P<0.02 | ++ |
| 400 | 31.3±1.1 | 99.1±18.3** | P<0.002 | ++ |
| 0(Normal control) | 30.0±0.5 | 47.7±3.5*** | P<0.001 | + |

| | | | | |
|---|---|---|---|---|
| a) Student's t-test (n=8). | | | | |

Weight gain in tumor bearing mice results from increased ascites retention with tumor cell growth.

As shown in Table 7, serum neutral fat in mice of the control group is remarkably higher than that of healthy mice. Comparison of feed intake between healthy and tumor bearing groups shows that feed intake in tumor bearing mice decreases day by day and that the energy balance turns negative around day 6 and after. This means that the cause of death by Ehrlich ascites tumor is starvation due to the feed intake energy exceeded by consumption. This suggests that excessive release of TNFα from tumor cells (a) enhances catabolic reaction, (b) weakens glucose assimilation by the expression of insulin resistance in peripheral tissue, and (c) promotes preferential consumption of glucose by tumor cells so that living bodies recruit muscle protein to compensate for energy deficiency and convert it into neutral fat in the liver to forward it to peripheral tissue.

In the groups treated with compound 1, however, the increase of serum neutral fat is dose-dependently suppressed and a significant increase in blood glucose level and a downward tendency in blood TNFα were also observed, showing that compound 1 inhibits tumor cells from producing TNFα.

## Claims

1. A nuclear receptor PPAR ligand, which is a compound selected from the group consisting of ascofuranone; and ascofuranone homologues or ascochlorin homologues having at least an orcylaldehyde moiety wherein the hydrogen of the hydroxyl group at the 2-position and/or 4-position of the orcylaldehyde moiety is substituted by a group selected from the group consisting of -(C₁-C₁₅)alkyl or alkenyl, aryl, (C₁-C₁₅)alkylaryl, -CH₂COOH, -CH₂COO(C₁-C₁₅)alkyl, -C(=O)(C₁-C₁₅)alkyl, -C(=O)aryl, -C(=O)(C₁-C₁₅)alkylaryl, -C(=O)(CH₂)₁₋ ₁₅COOH, nicotinoyl or isonicotinoyl.

2. The nuclear receptor PPAR ligand of Claim 1 wherein the compound is represented by the formula: 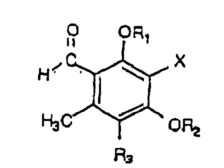 wherein X is 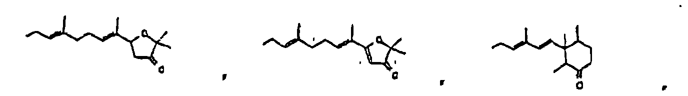 or 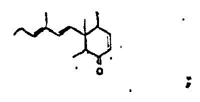 R₁ is H, -(C₁-C₅)alkyl or alkenyl or -C(=O)(C₁-C₅)alkyl; R₂ is H, -(C₁-C₅)alkyl or alkenyl, -CH₂COOH, -CH₂COO(C₁-C₅)alkyl, -C(=O)(C₁-C₅)alkyl, -C(=O)(CH₂)₁₋₅COOH, nicotinoyl or isonicotinoyl; and R₃ is H or Cl, provided that the case is excluded where X is 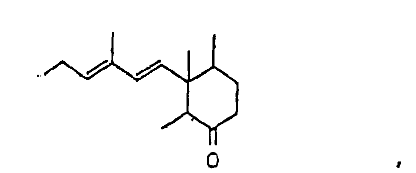 R₁ is H, R₂ is H and R₃ is Cl.

3. A compound represented by the formula: 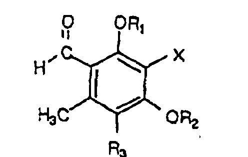 wherein X is 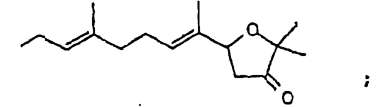 R₁ is H, -(C₁-C₅)alkyl or alkenyl or -C(=O)(C₁-C₅)alkyl; R₂ is H, -CH₂COOH, -CH₂COO(C₁-C₅)alkyl, -C(=O)(C₁-C₅)alkyl,-C(=O)(CH₂)₁₋₅COOH, nicotinoyl or isonicotinoyl; and R₃ is H or Cl, provided that the case is excluded where R₁ is H, R₂ is H and R₃ is Cl; or
wherein X is 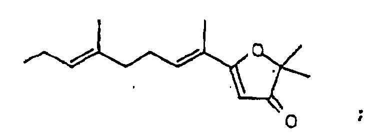 R₁ is H, -(C₁-C₅)alkyl or alkenyl or -C(=O)(C₁-C₅)alkyl; R₂ is H, -(C₁-C₅)alkyl or alkenyl, -CH₂COOH, -CH₂COO(C₁-C₅)alkyl, -C(=O)(C₁-C₅)alkyl, -C(=O)(CH₂)₁₋₅COOH, nicotinoyl or isonicotinoyl; and R₃ is H or Cl;
or a pharmaceutically acceptable salt thereof.

4. A compound represented by the formula: 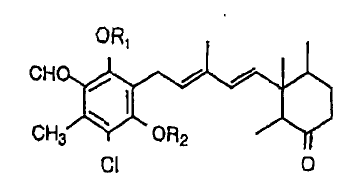 wherein R₁ is H, -(C₁-C₅)alkyl or alkenyl or -C(=O)(C₁-C₅)alkyl; and R₂ is H, -(C₁-C₅)alkyl, -CH₂COOH, -CH₂COO(C₁-C₅)alkyl, -C(=O)(C₁-C₅)alkyl, -C(=O)(CH₂)₁-₅COOH, nicotinoyl or isonicotinoyl, provided that the case is excluded where R₁ is H and R₂ is -CH₂COOH or nicotinoyl;
or a pharmaceutically acceptable salt thereof.

5. A nuclear receptor PPAR ligand, which is the compound of Claim 3 or 4.

6. A pharmaceutical composition for treating and/or preventing a disease or condition which can be alleviated by the PPAR ligand-dependent regulation of the transcription of genetic information, comprising the nuclear receptor PPAR ligand of Claim 1, 2 or 5 in an amount effective to treat and/or prevent said disease or condition and a pharmaceutically acceptable additive.

7. The pharmaceutical composition of Claim 6 wherein the disease or condition is diabetes including type II diabetes induced by the expression of insulin resistance; hypertension or cerebral angiopathy; arteriosclerosis including coronary arteriosclerosis; diabetic complication; chronic inflammation including chronic inflammation of organs including vessel, liver, kidney, joints, intestines and pancreas and chronic inflammation associated with autoimmune diseases; cachexia occurring in terminal cancer patients; gastrointestinal cancer including colon cancer, stomach cancer and liver cancer.

8. A composition for preventing degeneration and/or necrosis of β cells in the pancreatic islets of Langerhans to allow said cells to retain insulin producing function, comprising the nuclear receptor PPAR ligand of Claim 1, 2 or 5 in an amount effective to prevent degeneration and/or necrosis of β cells in the pancreatic islets of Langerhans and a pharmaceutically acceptable additive.

9. The composition of Claim 6, 7 or 8 wherein the additive is a synthetic aliphatic surfactant or a polymer substance soluble in organic solvents.

10. A method for treating and/or preventing a disease or condition which can be alleviated by the PPAR ligand-dependent regulation of the transcription of genetic information in a mammal, comprising administering to the mammal the compound of Claim 1, 2, 3 or 4 in an amount effective to treat and/or prevent said disease or condition optionally with a pharmaceutically acceptable additive.

11. The method of Claim 10 wherein the disease or condition is diabetes including type II diabetes induced by the expression of insulin resistance; hypertension or cerebral angiopathy; arteriosclerosis including coronary arteriosclerosis; diabetic complication; chronic inflammation including chronic inflammation of organs including vessel, liver, kidney, joints, intestines and pancreas and chronic inflammation associated with autoimmune diseases; cachexia occurring in terminal cancer patients; gastrointestinal cancer including colon cancer, stomach cancer and liver cancer.

12. A method for preventing degeneration and/or necrosis of β cells in the pancreatic islets of Langerhans in a mammal, comprising administering to the mammal the compound of Claim 1, 2, 3 or 4 in an amount effective to prevent degeneration and/or necrosis of β cells in the pancreatic islets of Langerhans optionally with a pharmaceutically acceptable additive.

13. The method of Claim 10, 11 or 12 wherein the additive is a synthetic aliphatic surfactant or a polymer substance soluble in organic solvents.

14. A use of the compound of Claim 1, 2, 3 or 4 for treating and/or preventing a disease or condition which can be alleviated by the PPAR ligand-dependent regulation of the transcription of genetic information in a mammal optionally with a pharmaceutically acceptable additive.

15. The use of Claim 14 wherein the disease or condition is diabetes including type II diabetes induced by the expression of insulin resistance; hypertension or cerebral angiopathy; arteriosclerosis including coronary arteriosclerosis; diabetic complication; chronic inflammation including chronic inflammation of organs including vessel, liver, kidney, joints, intestines and pancreas and chronic inflammation associated with autoimmune diseases; cachexia occurring in terminal cancer patients; gastrointestinal cancer including colon cancer, stomach cancer and liver cancer.

16. A use of the compound of Claim 1, 2, 3 or 4 for preventing degeneration and/or necrosis of β cells in the pancreatic islets of Langerhans in a mammal optionally with a pharmaceutically acceptable additive.

17. The use of Claim 14, 15 or 16 wherein the additive is a synthetic aliphatic surfactant or a polymer substance soluble in organic solvents.

18. A use of the compound of Claim 1, 2, 3 or 4 for preparing a drug for treating and/or preventing a disease or condition which can be alleviated by the PPAR ligand-dependent regulation of the transcription of genetic information in a mammal optionally with a pharmaceutically acceptable additive.

19. The use of Claim 18 wherein the disease or condition is diabetes including type II diabetes induced by the expression of insulin resistance; hypertension or cerebral angiopathy; arteriosclerosis including coronary arteriosclerosis; diabetic complication; chronic inflammation including chronic inflammation of organs including vessel, liver, kidney, joints, intestines and pancreas and chronic inflammation associated with autoimmune diseases; cachexia occurring in terminal cancer patients; gastrointestinal cancer including colon cancer, stomach cancer and liver cancer.

20. A use of the compound of Claim 1, 2, 3 or 4 for preparing a drug for preventing degeneration and/or necrosis of β cells in the pancreatic islets of Langerhans in a mammal optionally with a pharmaceutically acceptable additive.

21. The use of Claim 18, 19 or 20 wherein the additive is a synthetic aliphatic surfactant or a polymer substance soluble in organic solvents.

22. A Schiff base of a compound capable of functioning as a nuclear receptor PPAR ligand and an amino group of a serum protein, wherein said compound is the compound of Claim 1, 2, 3 or 4.
